# EUROPEAN PATENT APPLICATION

(11) **EP 2 535 063 A1**
(43) Date of publication of application: **19.12.2012**
(21) Application number: 12005958.9
(22) Date of filing: 08.08.2008
(51) Int. Cl.: A61L 27/38, A61L 27/54, A61L 27/58

(54) **Materials and methods for treating skeletal system damage and promoting skeletal system repair and regeneration**

(30) Priority: 08.08.2007 US 963929 P; 25.02.2008 US 66933 P
(62) Divisional of application: 08795156.2
(71) Applicant: Pervasis Therapeutics, Inc., Cambridge, Massachusetts 02139 (US)
(72) Inventor: Nugent, Helen, Marie, Needham, MA 02492 (US); Ng, Yin Shan, North Billerica, MA 01862 (US); Tjin, Tham Sjin Robert, Framingham, MA 01701 (US); Schubert, Shai, Sommerville, MA 02143 (US); Birkhead, James Richard, Westborough, MA 01581 (US)
(74) Representative: Lock, Graham James

(57) **Abstract**

Disclosed herein are materials and methods suitable for treating injured, damaged or diseased mineralized and non-mineralized skeletal tissues, including bones, joints, tendons, ligaments, cartilage and/or other non-mineralized skeletal tissues. The affected structure can be treated by contacting a surface of the skeletal element at or adjacent to or in the vicinity of an area of injury, damage or disease with an implantable material. The implantable material comprises a biocompatible matrix and cells and is in an amount effective to treat the affected structure. A composition comprising a biocompatible matrix and cells engrafted therein or thereon can be used to treat the affected structure. The composition can be a flexible planar material or a flowable composition.

## Description

### Background of the Invention

Skeletal system disorders include injuries, diseases or disorders that cause deviation from or interruption of the normal structure, function or connectivity of bones, joints, tendons, ligaments and cartilage. Skeletal system disorders can cause pain, discomfort or other problems, and many lead to serious medical conditions such as severe pain, disability, arthritis, and loss of mobility and functionality.

Current treatments for injured, damaged or diseased bones and other skeletal system disorders are limited and often have adverse consequences. Treatment options vary with age, health, and the severity of the injury or disease. One objective of the present invention is to provide methods and materials for the treatment of injured, damaged or diseased skeletal tissues, mineralized and non-mineralized. That is, the present invention provides methods and materials for treating injured, damaged or diseased bones, joints, tendons, ligaments, cartilage, and/or other non-mineralized skeletal tissue (collectively, "skeletal elements") and to promote repair and regeneration of same.

### Summary of the Invention

The present invention exploits the discovery that injured, damaged or diseased mineralized and non-mineralized skeletal tissues, including bones, joints, tendons, ligaments, cartilage and/or other non-mineralized skeletal tissues can be treated effectively by administration of a cell-based therapy to a surface of a site of injury, damage or disease of the affected structure. As disclosed herein, an implantable material comprising cells, preferably endothelial cells or cells having an endothelial-like phenotype, can be used to treat and manage injured, damaged or diseased mineralized or non-mineralized skeletal tissues including bones, joints, tendons, ligaments, cartilage and/or other non-mineralized skeletal tissues when the material is situated at or near the surface of the injured, damaged or diseased affected structure. This discovery permits the clinician to intervene in the treatment of an injured, damaged or diseased bone, joint, tendon, ligament, cartilage and/or other non-mineralized skeletal tissue for which there have heretofore been limited treatment options.

In one aspect, the invention is directed to a method of treating a skeletal system disorder in an individual in need thereof, the method comprising contacting with an implantable material a surface of a skeletal element at or adjacent to or in the vicinity of an area of injury, damage or discase wherein the implantable material comprises a biocompatible matrix and cells and further wherein the implantable material is in an amount effective to treat the skeletal system disorder in said individual.

In another aspect, the invention is directed to a method of treating a skeletal element in an individual in need thereof, the method comprising contacting with an implantable material a surface of a skeletal element at or adjacent to or in the vicinity of an area of surgical intervention, wherein said implantable material comprises a biocompatible matrix and cells and further wherein said implantable material is in an amount effective to treat the skeletal element in said individual.

In another aspect, the invention is directed to a composition suitable for the treatment or management of a skeletal element, the composition comprising a biocompatible matrix and cells, wherein the composition is in an amount effective to treat or manage the skeletal element.

According to various embodiments, the skeletal system disorder is a bone fracture, osteoporosis, Paget's disease of the bone, nonunion fracture, damaged ligament, damaged tendon, tendon graft, bone graft or cartilage injury. The skeletal element is a bone, tendon, ligament, cartilage, joint, mineralized skeletal tissue or non-mineralized skeletal tissue according to various embodiments.

In additional embodiments, the biocompatible matrix is a flexible planar material or a flowable material. The cells are endothelial cells, epithelial cells, endothelial-like cells, epithelial-like cells, non-endothelial cells or analogs thereof. According to another embodiment, the cells are a co-culture of at least two different cell types. According to several embodiments, the implantable material is applied to a surface of the skeletal element to promote bone repair, tendon repair, ligament repair or cartilage repair. According to an additional embodiment, the flowable composition further comprises an attachment peptide and the cells are engrafted on or to the attachment peptide. The composition, according to different embodiments, further includes a second therapeutic agent, an agent that inhibits infection, an anti-inflammatory agent, an attachment peptide, a transforming growth factor, a bone morphogenic protein, a cartilage-derived morphogenic protein, or a growth differentiation factor.

### Brief Description of the Drawings

Figures 1A and 1B are representative cell growth curves according to an illustrative embodiment of the invention.

Figure 2 is a graphical representation of the relative expression levels of ostopontin and bone sialoprotein in osteoblasts at 24 hours post treatment according to an illustrative osteoblast differentiation assay.

Figure 3 is a graphical representation of mouse osteoblast staining with and without TNFa or the implantable material according to an illustrative embodiment of the invention.

Figure 4 is a graphical representation of mouse chondrocyte staining with and without TNFa or the implantable material according to an illustrative embodiment of the invention.

Figure 5 is a graphical representation of the suppression of IL-1α-mediated cartilage damage by media conditioned by the implantable materials according to an illustrative cartilage plug assay.

Figure 6 is a graphical representation of enhanced GAG production and accumulation by primary porcine chondrocytes treated with conditioned media from the implantable material according to an illustrative primary chondrocyte functional assay.

### Detailed Description of the Invention

As explained herein, the invention is based on the discovery that a cell-based therapy can be used to treat, ameliorate, manage and/or reduce the effects of skeletal system disorders affecting mineralized and non-mineralized skeletal tissues, including, without limitation, injured, damaged or diseased bones or other components associated with the skeletal system including joints, tendons, ligaments, cartilage and/or other non-mineralized skeletal tissue (collectively, "skeletal elements"). The teachings presented below provide sufficient guidance to make and use the materials and methods of the present invention, and further provide sufficient guidance to identify suitable criteria and subjects for testing, measuring, and monitoring the performance of the materials and methods of the present invention.

When used in an effective amount, the cell-based therapy of the present invention, an implantable material comprising cells engrafted on, in and/or within a biocompatible matrix and having a preferred phenotype, produces factors positively associated with the proper regulation of bone formation, resorption and repair, and the formation and repair of other skeletal elements. For example, when used in an effective amount, the cells of the implantable material, when engrafted in or within a biocompatible matrix and having a preferred phenotype, can produce quantifiable amounts of heparan sulfate (HS), heparan sulfate proteoglycans (HSPGs), nitric oxide (NO), transforming growth factor-beta (TGF-β), fibroblast growth factors (FGFs) including basic fibroblast growth factor (bFGF), matrix metalloproteinases (MMPs) and/or tissue inhibitors of matrix metalloproteinases (TIMPs).

For example, the TGF-β1 isoform of TGF-β is involved in keeping an appropriate balance between bone resorption and bone formation. TGF-β1 is highly expressed during fracture healing, suggesting that its role extends to the process of bone repair. TGF-β is also involved in almost all stages of tendon healing. TGF-β is involved in stimulation of extrinsic cell migration, regulation of proteinases and fibronectin binding interactions, termination of cell proliferation through cyclin-dependent kinase inhibitors, and stimulation of collagen production. However, too high a level of certain TGF-β isoforms may be detrimental to tendon healing. For example, TGF-β1 is involved in tendon adhesion formation, which can significantly decrease the range of motion of a tendon. Therefore, the proper regulation of TGF-β activity is important for tendon healing.

BMPs form a unique group of proteins within the TGF-β superfamily. There is extensive evidence supporting the role of BMPs as regulators of bone induction, maintenance and repair. BMPs are highly expressed during fracture healing and have the ability to induce bone formation by regulating osteoblast differentiation and function. BMPs are expressed in the cells of developing bones in vivo, as well as in the fracture callus. BMP-2, -4, -3, -5, -6 and -7 have been shown to be important regulators of skeletal tissue formation and repair. Cartilage-derived morphogenic proteins (CDMP-1, -2 and -3; also known as BMP-14, -13, -12), a BMP subgroup, are essential for the formation of cartilaginous tissue during early limb development and for the formation of the articular joint cavity during joint development. CDMPs are also thought to play a role in the maintenance and regeneration of the articular cartilage. BMP-2 and BMP-7, have been shown to successfully unite critical sized defects in long bones. BMP-2 and BMP-7 also play a role in promotion of tendon-bone healing. Additionally, certain growth differentiation factors (GDF), such as but not limited to GDF-5, play a role in skeletal tissue growth, repair and maintenance.

As yet another example, MMPs and their respective inhibitors, TIMPs, are involved in the fracture healing process. New bone formation during fracture healing is mainly attributable to endochondral ossification preceded by soft callus formation, a process requiring extensive enzymatic remodeling of extracellular matrix (ECM) substrates that is mostly performed by MMPs. Scrum MNP-1 and -2 as well as TIMP-I and -2 have been shown to be significantly altered during the fracture healing process. An altered balance of the MMP/TIMP system in favor of proteolytic activity may be involved in the processes leading to fracture nonunion.

As yet other examples, HS and NO both play a role in fracture healing. HS is involved in bone repair by recruiting and enhancing the production of endogenous growth factors to the site of injury. It also enhances BMP-induced osteoclast differentiation by inhibiting BMP degredation. HS may also enhance growth factor activity within the callus of healing fractures to increase the expression of osteoblast genes important for osteogenesis. NO is important in fracture healing because it inhibits osteoclast activity and precursor recruitment, thereby having a supprcssive effect on bone resorption. NO is expressed during fracture healing, and suppression of nitric oxide synthase (NOS) impairs fracture healing. Furthermore, addition of an NO supplement after NOS inhibition can reverse impaired healing caused by NOS suppression. The endothelial nitric oxide synthetase (eNOS) pathway plays an essential role in regulating bone mass and bone turnover by modulating osteoblast function. It is thought that NO might enhance fracture healing, especially in situations where fracture healing is impaired due to other causes, for example malnutrition.

As a final example, bFGF, which is markedly up-regulated throughout tendon repair, is a potent stimulator of angiogenesis, cellular migration and proliferation. bFGF is thought to promote the formation of early repair tissue and to boost the initial stages of healing. However, it is thought this initial boost sets in motion a cascade of other stimuli which results in greatly improved fracture healing. Studies show that disruption of the gene for bFGF results in decreased osteoblast replication, decreased mineralized nodule formation and decrease new bone formation.

Accordingly, administration of an effective amount of the implantable material of the present invention can be used to treat, ameliorate, manage and/or reduce the effects of injured, damaged or diseased bones, joints, tendons, ligaments, and/or cartilage by providing a targeted supply of therapeutic factors *in vivo* in an amount sufficient to induce and/or manage healing of injured, damaged or diseased skeletal elements, for example, bone fractures.

Bone Injury: Bone injuries, damage or disease can be managed and repair promoted with the implantable material of the present invention. There are many conditions that can cause bone injury. Bones can be damaged by physical injury or progressive disease. Bone damage can be caused by falls or other trauma which cause a fracture or break in the bone. Diseases such as osteoporosis, and Paget's disease of the bone ("Paget's Disease") cause weakened bone structures, and lead to a serious risk of bone fractures. A fall or other trauma can cause injuries to the tendons and ligaments, injuries that frequently require surgical intervention to repair the defect, and may involve the attachment of a tendon or ligament graft to the bone. Physical injury from a fall or other trauma may also result in damaged cartilage. Damage to cartilage surrounding the bone can also be caused by osteoarthritis.

The Bone Healing Process: Bone has the unusual capacity to heal with its own tissue rather than with scar tissue. Bone healing occurs in three phases: the inflammatory phase, the repair phase and the remodeling phase. The bone healing process begins by the formation of a hematoma within the fracture site during the first few hours and days. Inflammatory cells infiltrate the bone, resulting in the formation of granulation tissue, ingrowth of vascular tissue, and migration of mesenchymal cells. During the repair phase, fibroblasts begin to lay down a stroma that supports vascular ingrowth. As vascular ingrowth progresses, a collagen matrix is laid down, osteoid is secreted and subsequently mineralized, leading to the formation of a soft callus around the repair site. Eventually, the soft callus ossifies, forming a bridge of woven bone between the fracture fragments. Fracture healing is completed during the remodeling stage in which the healing bone is restored to its original shape, structure and mechanical strength. The bone healing process is influenced by a variety of biochemical, biomechanical, cellular, hormonal and pathological mechanisms. As discussed above, many of the proteins and growth factors found in the implantable material assist in the bone healing process.

Bone Fractures: Bone fractures can be managed and repair promoted with the implantable material of the present invention. The most common cause of fractures is due to trauma. However, especially in the elderly, fractures often occur where the bone has been weakened by an underlying process such as osteoporosis, Paget's disease or tumors. Treatment of a bone facture focuses on reduction of the fracture to maintain proper alignment in order for the bone to heal properly. Current methods of treatment vary depending on the type and location of the fracture, the seriousness of the injury, the condition and needs of the patient. In most cases, reduction of a fracture involves cast immobilization. Traction may also be used as a preliminary treatment to achieve proper alignment. A functional cast or brace that allows movement of the nearby joints may also be used for certain types of fractures. For more serious fractures, open reduction and internal fixation may be used to reposition the bones into correct alignment and hold them together with pins, plates, screws or rods. Bone fragments can also be aligned by external fixation in which pins or screws are placed into the broken bone above and below the fracture site and are connected to a metal bar or bars outside the skin. If examination of the fracture shows that a quantity of bone has been lost as a result of the fracture, especially if there is a gap between the broken bone ends, a bone graft may be necessary to avoid delayed healing.

Complications of fracture treatment include malunion and nonunion. Malunion is the healing ofa fracture in an improper position. Malunion commonly results from poor alignment of the original fracture or drifting of previously well positioned bones. Additionally, shortening can develop as fractures with multiple fragments and poor quality bone undergo a gradual collapse. Nonunion is the failure of fracture fragments to unite or heal. Some of the causes of nonunion fractures include: insufficient or improper immobilization, infection, the presence of soft tissue interposed between the edges of the fractured bone, inadequate blood supply to the fracture site. Additionally, fractures that are open, comminuted, segmental, or pathologic are at higher risk of being nonunion fractures. As discussed above, the implantable material of the present invention provides quantifiable amounts of the growth factors that play a role in fracture healing.

Tendon and Ligament Injuries: Tendon and ligament injuries, damage or disease can be managed and repair promoted with the implantable material of the present invention. Tendons and ligaments connect muscle to bone and bone to bone, respectively. Tendon and ligament injuries can occur in anyone, but arc most common in athletes. Ligaments and tendons generally have a poor blood supply, leading to incomplete healing after injury. Frequently, injuries to tendons and ligaments require surgical treatment. For example, reconstruction of the anterior cruciate ligament (ACL) usually involves use of a tendon graft that is transplanted into bone tunnels at femoral and tibial insertion sites. Tendon-to-bone healing is important for the success of these grafts, however, tendon-to-bone healing is often slow or ineffective, necessitating a lengthy delay in returning to complete function. Repair of a torn Achilles tendon requires surgery to re-attach the tendon to itself, and patients are required to wear a boot or a cast for a lengthy period of time post-surgery to allow the tendon to heal. Rotator cuff injuries have a variety of treatment options depending on the severity of the injury. While a partial tear may require only debridement, a complete tear within the substance of the tendon is repaired by suturing the two sides of the tendon. If the tendon is torn from its insertion on the greater tuberosity of the humerus, it will be repaired directly to bone. Effective healing of a tendon or tendon graft to itself or to the bone is essential to the success of these surgeries. Because tendon and ligament injuries frequently occur in athletes who have a need to return to full function as quickly as possible, a method of accelerated healing is needed. As mentioned above, some of the growth factors thought to be important in tendon and ligament healing are found in the implantable material of the present invention. The present invention is particularly well-suited for treatment of bone attachment sites at which a functional connection to a non-mineralized tissue is required.

Osteoporosis: Osteoporosis can be managed with the implantable material of the present invention. Osteoporosis is a disease of progressive bone loss that is associated with an increased risk of fractures. Osteoporosis often has no signs or symptoms, and goes unnoticed until a fracture occurs. It often causes a loss of height and dowager's hump. It is estimated that fractures due to osteoporosis occur in one in two women and one in five men over the age of 65. Osteoporosis can lead to fractures in any bone but may cause serious fractures in the hip or spine. Hip fractures, especially in older adults, can result in disability and even death from postoperative complications. Compression fractures of the spine can cause severe pain and require a long recovery, and compression fractures can lead to the loss of several inches in height.

Current treatment of osteoporosis focuses on the prevention of further bone loss, prevention of falls and pain management. Patients are encouraged to make certain lifestyle changes, such as maintaining a diet with adequate calcium and vitamin D, and regular weight bearing exercise, with the goal of increasing bone strength and preventing the progression of the disease. Many medications are currently used to prevent and treat osteoporosis. In women, osteoporosis is linked to decreased estrogen levels after menopause, and estrogen replacement therapy can be used to prevent further bone loss. However, estrogen replacement therapy has also been linked to an increased risk of certain other conditions such as breast cancer and coronary heart disease, therefore, it is inappropriate for all women. Selective estrogen receptor modulators (SERMs) that mimic the effects of estrogen have been used to prevent bone loss without the increased risks of estrogen replacement therapy. Biophosphonates and calcitonin have also been shown to be effective at decreasing further bone loss. Vertebroplasty, a surgical procedure to treat small fractures in the spinal column due to osteoporosis, can also be performed. Because of these limited treatment options, osteoporosis patients would benefit from methods to restore proper bone formation. The implantable material of the present invention contains growth factors that help restore proper bone formation.

Paget's Disease of the Bone: Paget's disease can be managed with the implantable material of the present invention. Paget's disease of the bone is caused by a disruption in the normal process of bone remodeling. In normal bone remodeling, osteoclasts absorb old bone and osteoblasts make new bone to replace the old bone. In Paget's disease patients, osteoclasts are more active than osteoblasts. In other words, there is more bone adsorption than normal. As a result, osteoblasts overproduce new bone, but the new bone that is made is abnormally large, deformed, and does not fit together correctly. The new bone produced in Paget's disease patients is large and dense, but also weak and brittle, and as such it is prone to fractures, bowing and deformities.

Paget's disease usually affects the skull, spine, and the bones in the arms legs and pelvis. The disease may he present in only one or two areas of the body, or it may be widespread. Signs and symptoms of Paget's disease include bone pain, joint pain, sciatica, numbness, tingling, weakness, hearing loss and double vision. However, Paget's disease often has no symptoms and it is diagnosed by x-ray or bone scan after injury or when performing these tests for other reasons. Increased alkaline phosphatase levels may be indicative of Paget's disease.

Current treatment for Paget's disease focuses on pain management, prevention of falls, diet, exercise and prevention of further progression of the disease. Biophosphonates block osteoclasts and can be effective against further progression. Calcitonin is also an available treatment. In rare cases, surgery is required to help fractures heal, to replace joints damaged by severe arthritis or to realign fractured bones. However, patients with Paget's disease are at risk for serious blood loss during surgery due to the hypervascularity caused by the disease. Paget's disease patients would benefit from methods that can regulate the activity of osteoblasts and osteoclasts to restore normal bone formation and resorption. The implantable material of the present invention contains factors that regulate the activity of osteoblasts and osteoclasts.

Cartilage Injuries: Like tendon and ligament injuries, cartilage injuries commonly occur in athletes. Since cartilage is needed for effective joint movement, a cartilage injury can cause serious symptoms that affect patients' ability to function. Cartilage injuries cause symptoms such as locking, catching, localized pain and swelling, loss of range of motion, and can lead to weakness of the affected joint. Because cartilage has minimal ability to heal on its own, surgical procedures are often necessary to stimulate new cartilage growth. Because of the limited ability for cartilage to repair itself, patients with cartilage injuries or diseases would benefit from methods to control breakdown of cartilage and to restore cartilage formation. The implantable material of the present invention contains growth factors that can stimulate cartilage formation. Chondral as well as osteochondral defects can be treated in accordance with the teachings disclosed herein.

Rheumatoid Arthritis: Rheumatoid arthritis can be managed with the implantable material of the present invention. Rheumatoid arthritis is a chronic autoimmune disorder that causes inflammation of the synovial membrane that covers the joints and can lead to substantial loss of function and mobility. As the condition progresses, the synovial membrane inflammation can lead to erosion and destruction of the joint surfaces, which impairs the joint range of motion. Because of the limited ability for synovial membranes and joint surfaces to repair themselves, patients with rheumatoid arthritis would benefit from methods to control inflammation of synovial membranes and to restore synovial membrane and joint surface formation. The implantable material of the present invention contains growth factors that can stimulate synovial membrane and joint surface repair and formation.

Osteoarthritis: Osteoarthritis can be managed with the implantable material of the present invention. Osteoarthritis is a condition in which low-grade inflammation caused by abnormal wearing of the cartilage that covers and acts as a cushion inside joints often combined with destruction of synovial fluid that lubricates these joints results in pain in the joints. Because of the limited ability for cartilage and synovial membranes to repair themselves, patients with osteoarthritis would benefit from methods to control inflammation and destruction of cartilage and synovial membranes. The implantable material of the present invention contains growth factors that can stimulate cartilage and synovial membrane repair and formation.

The materials and methods of the present invention can be used in connection with any of the above-described injuries, damage and diseases, or numerous other mineralized or non-mineralized skeletal tissue injuries, damage or diseases including bone, joint, tendon, ligament or cartilage diseases. In addition, the materials and methods of the present invention can be used in connection with any surgical intervention to correct defects in bone, joints, tendon, ligament, cartilage or other non-mineralized skeletal tissue injury or to treat disease. The materials and methods of the present invention can be used in conjunction with these or other surgeries to increase effectiveness and promote healing. Other skeletal tissues susceptible to treatment with the present invention include intervertebral discs, menisci, synovial membranes, synovial capsule and avascular skeletal tissue.

### Implantable Material

General Considerations: The implantable material of the present invention comprises cells engrafted on, in and/or within a biocompatible matrix. Engrafted means securedly attached via cell to cell and/or cell to matrix interactions such that the cells meet the functional or phenotypical criteria set forth herein and withstand the rigors of the preparatory manipulations disclosed herein. As explained elsewhere herein, an operative embodiment of implantable material comprises a population of cells associated with a supporting substratum, preferably a differentiated cell population and/or a near-confluent, confluent or post-confluent cell population, having a preferred functionality and/or phenotype.

Complex substrate specific interactions regulate the intercellular morphology and secretion of the cells and, accordingly, also regulate the functionality and phenotype of the cells associated with the supporting substratum. Cells associated with certain preferred biocompatible matrices, contemplated herein, may grow and conform to the architecture and surface of the local struts ofmatrix pores with less straining as they mold to the matrix. Also, the individual cells of a population of cells associated with a matrix retain distinct morphology and secretory ability even without complete contiguity between the cells. Further, cells associated with a biocompatible matrix may not exhibit planar restraint, as compared to similar cells grow as a monolayer on a tissue culture plate.

It is understood that embodiments of implantable material likely shed cells during preparatory manipulations and/or that certain cells are not as securely attached as are other cells. All that is required is that implantable material comprises cells associated with a supporting substratum that meet the functional or phenotypical criteria set forth herein.

That is, interaction between the cells and the matrix during the various phases of the cells' growth cycle can influence the cells' phenotype, with the preferred inhibitory phenotype described elsewhere herein correlating with quiescent cells (i.e., cells which are not in an exponential growth cycle). As explained elsewhere herein, it is understood that, while a quiescent cell typifies a population of cells which are near-confluent, confluent or post-confluent, the inhibitory phenotype associated with such a cell can be replicated by manipulating or influencing the interaction between a cell and a matrix so as to render a cell quiescent-like.

The implantable material of the present invention was developed on the principals of tissue engineering and represents a novel approach to addressing the above-described clinical needs. The implantable material of the present invention is unique in that the viable cells engrafted on, in and/or within the biocompatible matrix are able to supply to the mineralized or non-mineralized skeletal tissue, including bone, joint, tendon, ligament, cartilage and/or other non-mineralioed skeletal tissue, multiple cell-based products in physiological proportions under physiological feed-back control. As described elsewhere herein, the cells suitable for use with the implantable material include endothelial, endothelial-like, non-endothelial cells or analogs thereof. Local delivery of multiple compounds by these cells in a physiologically-dynamic dosing provide more effective regulation of the processes responsible for maintaining functional bone, joint, tendon, ligament, cartilage and/or other non-mineralized skeletal tissue structures and diminishing the clinical sequel associated with injury, damage or disease of skeletal elements.

The implantable material of the present invention, when wrapped, deposited adjacent to or otherwise contacted with the surface of a injured, damaged or diseased bone, joint, tendon, ligament, cartilage and/or other non-mineralized skeletal tissue site serves to reestablish homeostasis. That is, the implantable material of the present invention can provide an environment which mimics supportive physiology and is conducive to manage and/or promote healing a site of injury, damage or disease of these skeletal elements.

For purposes of the present invention, contacting means directly or indirectly interacting with a surface of a bone, joint, tendon, ligament, cartilage and/or other non-mineralized skeletal tissue as defined elsewhere herein. As used herein, the term "surface" includes the site of a skeletal element that is exposed due to injury damage or disease, e.g. the surface of the fracture site in a bone fracture. In the case of certain preferred embodiments, actual physical contact is not required for effectiveness. In other embodiments, actual physical contact is preferred. All that is required to practice the present invention is exterior deposition of an implantable material at, adjacent to or in the vicinity of an injured, diseased or damaged bone, joint, tendon, ligament, cartilage and/or other non-mineralized skeletal tissue in an amount effective to treat the injured or diseased site. In the case of certain diseases or injuries, a diseased or injured site can clinically manifest on an interior surface. In the case of other diseases or injuries, a diseased or injured site can clinically manifest on a surface of the structure. In some diseases or injuries, a diseased or injured site can clinically manifest on both an interior surface and a surface of the structure. The present invention is effective to treat any of the foregoing clinical manifestations.

For example, endothelial cells can release a wide variety of agents that have been shown to promote bone, tendon and ligament healing. As exemplified herein, a composition and method of use that recapitulates normal physiology and dosing is useful to promote bone healing, Typically, treatment includes placing the implantable material of the present invention at, adjacent to or in the vicinity of the injured, damaged or diseased bone. When wrapped, wrapped around, deposited, or otherwise contacting a bone, the cells of the implantable material can provide growth regulatory compounds to the bone. It is contemplated that, while in contact with the bone, the implantable material of the present invention comprising a biocompatible matrix or particle with engrafted cells provides a continuous supply of multiple regulatory and therapeutic compounds from the engrafted cells to the skeletal element.

Cell Source: As described herein, the implantable material of the present invention comprises cells. Cells can be allogeneic, xenogeneic or autologous. In certain embodiments, a source of living cells can be derived from a suitable donor. In certain other embodiments, a source of cells can be derived from a cadaver or from a cell bank.

In one currently preferred embodiment, cells are endothelial cells. In a particularly preferred embodiment, such endothelial cells are obtained from vascular tissue, preferably but not limited to arterial tissue. As exemplified below, one type of vascular endothelial cell suitable for use is an aortic endothelial cell. Another type of vascular endothelial cell suitable for use is umbilical cord vein endothelial cells. And, another type of vascular endothelial cell suitable for use is coronary artery endothelial cells. Yet another type of vascular endothelial cell suitable for use is saphenous vein endothelial cells. Yet other types of vascular endothelial cells suitable for use with the present invention include pulmonary artery endothelial cells and iliac artery endothelial cells.

In another currently preferred embodiment, suitable endothelial cells can be obtained from non-vascular tissue. Non-vascular tissue can be derived from any anatomical structure or can be derived from any non-vascular tissue or organ. Non-vascular tissue can be derived from other tissue types. Exemplary anatomical structures include structures of the vascular system, the renal system, the reproductive system, the genitourinary system, the gastrointestinal system, the pulmonary system, the respiratory system and the ventricular system of the brain and spinal cord.

In another embodiment, endothelial cells can be derived from endothelial progenitor cells or stem cells. In yet still another embodiment, endothelial cells can be derived from progenitor cells or stem cells generally. In other preferred embodiments, cells can be non-endothelial cells that are allogeneic, xenogeneic or autologous and can be derived from vascular, or other tissue or organ. Cells can be selected on the basis of their tissue source and/or their immunogenicity. Exemplary non-endothelial cells include epithelial cells, osteoblasts, ostcocytes, osteoclasts, fibroblasts, tenocytes, ligament cells, chondrocytes, secretory cells, smooth muscle cells, stem cells, bone stem cells, endothelial progenitor cells, cardiomyocytes, secretory and ciliated cells. The present invention also contemplates any of the foregoing which are genetically altered, modified or engineered.

In a further embodiment, two or more types of cells are co-cultured to prepare the present composition. For example, a first cell can be introduced into the biocompatible implantable material and cultured until confluent. The first cell type can include, for example, endothelial cells, epithelial cells, osteoblasts, osteocytes, osteoclasts, fibroblasts, tenocytes, ligament cells, chondrocytes, secretory cells, smooth muscle cells, stem cells, bone stem cells, endothelial progenitor cells, a combination of smooth muscle cells and fibroblasts, any other desired cell type or a combination of desired cell types suitable to create an environment conducive to growth of the second cell type. Once the first cell type has reached confluence, a second cell type is seeded on top of the first confluent cell type in, on or within the biocompatible matrix and cultured until both the first cell type and second cell type have reached confluence. The second cell type may include, for example, epithelial cells, osteoblasts, osteocytes, osteoclasts, fibroblasts, tenocytes, ligament cells, chondrocytes, secretory cells, smooth muscle cells, stem cells, bone stem cells, endothelial cells, endothelial progenitor cells, or any other desired cell type or combination of cell types. It is contemplated that the first and second cell types can be introduced step wise, or as a single mixture. It is also contemplated that cell density can be modified to alter the ratio of the first cell type to the second cell type.

To prevent over-proliferation of smooth muscle cells or another cell type prone to excessive proliferation, the culture procedure and timing can be modified. For example, following confluence of the first cell type, the culture can be coated with an attachment factor suitable for the second cell type prior to introduction of the second cell type. Exemplary attachment factors include coating the culture with gelatin to improve attachment of endothelial cells. According to another embodiment, heparin can be added to the culture media during culture of the second cell type to reduce the proliferation of the first cell type and to optimize the desired first cell type to second cell type ratio. For example, after an initial growth of smooth muscle cells, heparin can be administered to control smooth muscle cell growth to achieve a greater ratio of endothelial cells to smooth muscle cells.

All that is required of the cells of the present composition is that they exhibit one or more preferred phenotypes or functional properties. As described earlier herein, the present invention is based on the discovery that a cell having a readily identifiable phenotype when associated with a preferred matrix (described elsewhere herein) can facilitate, restore and/or otherwise modulate cell physiology and/or homeostasis associated with the treatment of injuries, damage or disease to skeletal elements.

For purposes of the present invention, one such preferred, readily identifiable phenotype typical of cells of the present invention is an ability to inhibit or otherwise interfere with smooth muscle cell proliferation as measured by the *in vitro* assays described below. This is referred to herein as the inhibitory phenotype.

One other readily identifiable phenotype exhibited by cells of the present composition is that they are able to inhibit abnormal fibroblast proliferation and/or migration and abnormal collagen deposition and/or accumulation. Fibroblast activity and collagen deposition activity can be determined using an *in vitro* fibroblast proliferation and/or an *in vitro* collagen accumulation assay, described below.

Another readily identifiable phenotype exhibited by cells of the present composition is that they are anti-thrombotic or are able to inhibit platelet adhesion and aggregation. Anti-thrombotic activity can be determined using an *in vitro* heparan sulfate assay and/or an *in vitro* platelet aggregation assay, described below.

A further readily identifiable phenotype exhibited by cells of the present composition is the ability to restore the proteolytic balance, the MMP-TIMP balance, the ability to decrease expression of MMPs relative to the expression of TIMPs, or the ability to increase expression ofTIMPs relative to the expression of MMPs. Proteolytic balance activity can be determined using an *in vitro* TIMP assay and/or an *in vitro* MMP assay described below.

In a typical operative embodiment of the present invention, cells need not exhibit more than one of the foregoing phenotypes. In certain embodiments, cells can exhibit more than one of the foregoing phenotypes.

While the foregoing phenotypes each typify a functional endothelial cell, such as but not limited to a vascular endothelial cell, a non-endothelial cell exhibiting such a phenotype(s) is considered endothelial-like for purposes of the present invention and thus suitable for use with the present invention. Cells that arc endothelial-like are also referred to herein as functional analogs of endothelial cells; or functional mimics of endothelial cells. Thus, by way of example only, cells suitable for use with the materials and methods disclosed herein also include stem cells or progenitor cells that give rise to endothelial-like cells; cells that are non-endothelial cells in origin yet perform functionally like an endothelial cell using the parameters set forth herein; cells of any origin which are engineered or otherwise modified to have endothelial-like functionality using the parameters set forth herein.

Typically, cells of the present invention exhibit one or more of the aforementioned functionalities and/or phenotypes when present and associated with a supporting substratum, such as the biocompatible matrices described herein. It is understood that individual cells attached to a matrix and/or interacting with a specific supporting substratum exhibit an altered expression of functional molecules, resulting in a preferred functionality or phenotype when the cells are associated with a matrix or supporting substratum that is absent when the cells are not associated with a supporting substratum.

According to one embodiment, the cells exhibit a preferred phenotype when the basal layer of the cell is associated with a supporting substratum. According to another embodiment, the cells exhibit a preferred phenotype when present in confluent, near confluent or post-confluent populations. As will be appreciated by one of ordinary skill in the art, populations of cells, for example, substrate adherent cells, and confluent, near confluent and post-confluent populations of cells, are identifiable readily by a variety of techniques, the most common and widely accepted of which is direct microscopic examination. Others include evaluation of cell number per surface area using standard cell counting techniques such as but not limited to a hemacytometer or coulter counter.

Additionally, for purposes of the present invention, endothelial-like cells include but are not limited to cells which emulate or mimic functionally and phenotypically the preferred populations of cells set forth herein, including, for example, differentiated endothelial cells and confluent, near confluent or post-confluent endothelial cells, as measured by the parameters set forth herein.

Thus, using the detailed description and guidance set forth below, the practitioner of ordinary skill in the art will appreciate how to make, use, test and identify operative embodiments of the implantable material disclosed herein. That is, the teachings provided herein disclose all that is necessary to make and use the present invention's implantable materials. And further, the teachings provided herein disclose all that is necessary to identify, make and use operatively equivalent cell-containing compositions. At bottom, all that is required is that equivalent cell-containing compositions are effective to treat, manage, modulate and/or ameliorate bone, joint, tendon, ligament, cartilage and/or other non-mineralized skeletal tissue injuries, damage or diseases in accordance with the methods disclosed herein. As will be appreciated by the skilled practitioner, equivalent embodiments of the present composition can be identified using only routine experimentation together with the teachings provided herein.

In certain preferred embodiments, endothelial cells used in the implantable material of the present invention are isolated from the aorta of human cadaver donors. Each lot of cells is derived from a single donor or from multiple donors, tested extensively for endothelial cell purity, biological function, the presence of bacteria, fungi, known human pathogens and other adventitious agents. The cells are cryopreserved and banked using well-known techniques for later expansion in culture for subsequent formulation in biocompatible implantable materials.

Cell Preparation: As stated above, suitable cells can be obtained from a variety of tissue types and cell types. In certain preferred embodiments, human aortic endothelial cells used in the implantable material are isolated from the aorta of cadaver donors. In other embodiments, porcine aortic endothelial cells (Cell Applications, San Diego, CA) are isolated from normal porcine aorta by a similar procedure used to isolate human aortic endothelial cells. Each lot of cells can be derived from a single donor or from multiple donors, tested extensively for endothelial cell viability, purity, biological function, the presence of mycoplasma, bacteria, fungi, yeast, known human pathogens and other adventitious agents. The cells are further expanded, characterized and cryopreserved to form a working cell bank at the third to sixth passage using well-known techniques for later expansion in culture and for subsequent formulation in biocompatible implantable material.

The human or porcine aortic endothelial cells are prepared in T-75 flasks pre-treated by the addition of approximately 15 ml of endothelial cell growth media per flask. Human aortic endothelial cells are prepared in Endothelial Growth Media (EGM-2, Cambrex Biosciences, East Rutherford, NJ). EGM-2 consists of Endothelial Cell Basal Media (EBM-2, Cambrex Biosciences) supplemented with EGM-2 singlequots, which contain 2% FBS. Porcine cells are prepared in EBM-2 supplemented with 5% FBS and 50 µg/ml gentamicin. The flasks are placed in an incubator maintained at approximately 37°C and 5% CO₂/95% air, 90% humidity for a minimum of 30 minutes. One or two vials of the cells are removed from the - 160°C to-140°C freezer and thawed at approximately 37°C. Each vial of thawed cells is seeded into two T-75 flasks at a density of approximately 3 x 10³ cells per cm², preferably, but no less than 1.0 x 10³ and no more than 7.0 x 10³; and the flasks containing the cells are returned to the incubator. After about 8-24 hours, the spent media is removed and replaced with fresh media. The media is changed every two to three days, thereafter, until the cells reach approximately 85-100% confluence preferably, but no less than 60% and no more than 100%. When the implantable material is intended for clinical application, only antibiotic-free media is used in the post-thaw culture of human aortic endothelial cells and manufacture of the implantable material of the present invention.

The endothelial cell growth media is then removed, and the monolayer of cells is rinsed with 10 ml of HEPES buffered saline (HEPES). The HEPES is removed, and 2 ml of trypsin is added to detach the cells from the surface of the T-75 flask. Once detachment has occurred, 3 ml of trypsin neutralizing solution (TNS) is added to stop the enzymatic reaction. An additional 5 ml of HEPES is added, and the cells are enumerated using a hemocytometer. The cell suspension is centrifuged and adjusted to a density of, in the case of human cells, approximately 2.0 - 1.75 x 10⁶ cells/ml using EGM-2 without antibiotics, or in the case of porcine cells, approximately 2.0 - 1.50 x 10⁶ cells/ml using EBM-2 supplemented with 5% FBS and 50 µg/ml gentamicin.

Biocompatible Matrix: According to the present invention, the implantable material comprises a biocompatible matrix. The matrix is permissive for cell growth and attachment to, on or within the matrix. The matrix is flexible and conformable. The matrix can be a solid, a semi-solid or flowable porous composition. For purposes of the present invention, flowable composition means a composition susceptible to administration using an injection or injection-type delivery device such as, but not limited to, a needle, a syringe or a catheter. Other delivery devices which employ extrusion, ejection or expulsion are also contemplated herein. Porous matrices are preferred. The matrix also can be in the form of a flexible planar form. The matrix also can be in the form of a gel, a foam, a suspension, a particle, a microcarrier, a microcapsule, or a fibrous structure. A preferred flowable composition is shape-retaining. A currently preferred matrix has a particulate form. The biocompatible matrix can comprise particles and/or microcarriers and the particles and/or microcarriers can further comprise gelatin, collagen, fibronectin, fibrin, laminin or an attachment peptide. One exemplary attachment peptide is a peptide of sequence arginine-glycine-aspartate (RGD).

The matrix, when implanted on a surface of a bone structure, can reside at the implantation site for at least about 7-90 days, preferably about at least 7-14 days, more preferably about at least 14-28 days, most preferably about at least 28-90 days before it bioerodes.

One preferred matrix is Gelfoam^{®} (Pfizer, Inc., New York, NY), an absorbable gelatin sponge (hereinafter "Gelfoam matrix"). Another preferred matrix is Surgifoam^{®} (Johnson & Johnson, New Brunswick, NJ), also an absorbable gelatin sponge. Gelfoam and Surgifoam matrices are porous and flexible surgical sponges prepared from a specially treated, purified porcine dermal gelatin solution.

According to another embodiment, the biocompatible matrix material can be a modified matrix material. Modifications to the matrix material can be selected to optimize and/or to control function of the cells, including the cells' phenotype (e.g., the inhibitory phenotype) as described above, when the cells are associated with the matrix. According to one embodiment, modifications to the matrix material include coating the matrix with attachment factors or adhesion peptides that enhance the ability of the cells to regulate smooth muscle cell and/or fibroblast proliferation and migration, to regulate collagen deposition, to regulate fibrosis, to regulate MMP and TIMP production, to regulate inflammation, to regulate heparan sulfate production, to regulate prostacyclin production, to regulate TGF-β₁ and nitric oxide (NO) production, and/or regulate bFGF production.

According to another embodiment, the matrix is a matrix other than Gelfoam. Additional exemplary matrix materials include, for example, fibrin gel, alginate, gelatin bead microcarriers, polystyrene sodium sulfonate microcarriers, collagen coated dextran microcarriers, PLA/PGA and pHEMA/MMA copolymers (with polymer ratios ranging from 1-100% for each copolymer). According to one embodiment, a synthetic matrix material, for example, PLA/PGA, is treated with NaOH to increase the hydrophilicity of the material and, therefore, the ability of the cells to attach to the material. According to a preferred embodiment, these additional matrices are modified to include attachment factors or adhesion peptides, as recited and described above. Exemplary attachment factors include, for example, gelatin, collagen, fibronectin, fibrin gel, and covalently attached cell adhesion ligands (including for example RGD) utilizing standard aqueous carbodiimide chemistry. Additional cell adhesion ligands include peptides having cell adhesion recognition sequences, including but not limited to: RGDY, REDVY, GRGDF, GPDSGR, GRGDY and REDV.

That is, these types of modifications or alterations of a substrate influence the interaction between a cell and a matrix which, in turn, can mediate expression of the preferred inhibitory phenotype described elsewhere herein. It is contemplated that these types of modifications or alterations of a substrate can result in enhanced expression of an inhibitory phenotype; can result in prolonged or further sustained expression of an inhibitory phenotype; and/or can confer such a phenotype on a cell which otherwise in its natural state does not exhibit such a phenotype as in, for example but not limited to, an exponentially growing or non-quiescent cell. Moreover, in certain circumstances, it is preferable to prepare an implantable material of the present invention which comprises non-quiescent cells provided that the implantable material has an inhibitory phenotype in accordance with the requirements set forth elsewhere herein. As already explained, the source of cells, the origin of cells and/or types of cells useful with the present invention are not limited; all that is required is that the cells express an inhibitory phenotype.

Embodiments of Implantable Materials: As stated earlier, implantable material of the present invention can be a flexible planar form or a flowable composition. When in a flexible planar form, it can assume a variety of shapes and sizes, preferably a shape and size which conforms to a contoured surface of a bone, tendon or ligament when situated at or adjacent to or in the vicinity of an injured or diseased site. Examples of preferred configurations suitable for use in this manner are disclosed in co-owned international patent application PCT/US05/43967 filed on December 6, 2005 (also known as Attorney Docket No. ELV-002PC), the entire contents of which are herein incorporated by reference.

Flowable Composition: In certain embodiments contemplated herein, the implantable material of the present invention is a flowable composition comprising a particulate biocompatible matrix which can be in the form of a gel, a foam, a suspension, a particle, a microcarrier, a macrocarrier, a microcapsule, macroporous beads, or other flowable material. The current invention contemplates any flowable composition that can be administered with an injection-type delivery device. For example, a delivery device such as a percutaneous injection-type delivery device is suitable for this purpose as described below. The flowable composition is preferably a shape-retaining composition. Thus, an implantable material comprising cells in, on or within a flowable-type particulate matrix as contemplated herein can be formulated for use with any injectable delivery device ranging in internal diameter from about 18 gauge to about 30 gauge and capable of delivering about 50 mg of flowable composition comprising particulate material containing preferably about 1 million cells in about 1 to about 3 ml of flowable composition.

According to a currently preferred embodiment, the flowable composition comprises a biocompatible particulate matrix such as Gelfoam^{®} particles, Gelfoam^{®} powder, or pulverized Gelfoam^{®} (Pfizer Inc., New York, NY) (hereinafter "Gelfoam particles"), a product derived from porcine dermal gelatin. According to another embodiment, the particulate matrix is Surgifoam^{™} (Johnson & Johnson, New Brunswick, NJ) particles, comprised of absorbable gelatin powder. According to another embodiment, the particulate matrix is Cytodex-3 (Amersham Biosciences, Piscataway, NJ) microcarriers, comprised of denatured collagen coupled to a matrix of cross-linked dextran. According to a further embodiment, the particulate matrix is CultiSpher-G (Percell Biolytica AB, Astorp, Sweden) microcarrier, comprised of porcine gelatin. According to another embodiment, the particulate matrix is a macroporous material. According to one embodiment, the macroporous particulate matrix is CytoPore (Amersham Biosciences, Piscataway, NJ) microcarrier, comprised of cross-linked cellulose which is substituted with positively charged N,N,-diethylaminoethyl groups.

According to alternative embodiments, the biocompatible implantable particulate matrix is a modified biocompatible matrix. Modifications include those described above for an implantable matrix material.

Related flowable compositions suitable for use to manage the development and/or progression of healing of skeletal elements in accordance with the present invention are disclosed in co-owned international patent application PCT/US05/43844 filed on December 6, 2005 (also known as Attorney Docket No. ELV-009PC), the entire contents of which are herein incorporated by reference.

Preparation of Implantable Material: Prior to cell seeding, the biocompatible matrix is re-hydrated by the addition of water, buffers and/or culture media such as EGM-2 without antibiotics at approximately 37°C and 5% CO₂/95% air for 12 to 24 hours. The implantable material is then removed from their rehydration containers and placed in individual tissue culture dishes. The biocompatible matrix is seeded at a preferred density of approximately 1.5-2.0 x 10⁵ cells (1.25-1.66 x 10⁵ cells /cm³ of matrix) and placed in an incubator maintained at approximately 37°C and 5% CO₂/95% air, 90% humidity for 3-4 hours to 24 hours to facilitate cell attachment. The seeded matrix is then placed into individual containers (Evergreen, Los Angeles, CA) or tubes, each fitted with a cap containing a 0.2 µm filter with EGM-2 and incubated at approximately 37°C and 5% CO₂/95% air. Alternatively, three seeded matrices can be placed in 150 mL bottles. The media is changed every two to three days, thereafter, until the cells have reached near-confluence, confluence or post-confluence. The cells in one preferred embodiment are preferably passage 6, but cells of fewer or more passages can be used.

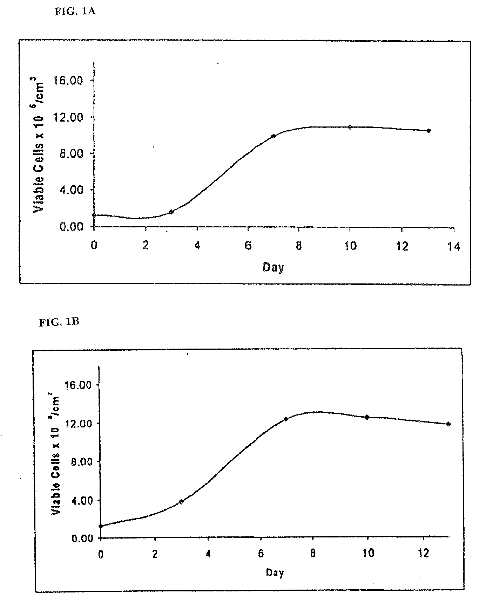

Cell Growth Curve and Confluence: A sample of implantable material is removed on or around days 3 or 4, 6 or 7, 9 or 10, and 12 or 13, the cells are counted and assessed for viability, and a growth curve is constructed and evaluated in order to assess the growth characteristics and to determine whether confluence, near confluence or post-confluence has been achieved. Representative growth curves from two preparations of implantable material comprising porcine aortic endothelial cell implanted lots are presented in FIGS. 1A and 1B. In these examples, the implantable material is in a flexible planar form. Generally, one of ordinary skill will appreciate the indicia of acceptable cell growth at early, mid- and late time points, such as observation of an increase in cell number at the early time points (when referring to FIG. 1A, between about days 2-6), followed by a near confluent phase (when referring to FIG. 1A, between about days 6-8), followed by a plateau in cell number once the cells have reached confluence as indicated by a relatively constant cell number (when referring to FIG. 1A, between about days 8-10) and maintenance of the cell number when the cells are post-confluent (when referring to FIG. 1A, between about days 10-14). For purposes of the present invention, cell populations which are in a plateau for at least 72 hours are preferred.

Cell counts are achieved by complete digestion of the aliquot of implantable material such as with a solution of 0.5 mg/ml collagenase in a CaCl₂ solution in the case of gelatin-based matrix materials. After measuring the volume of the digested implantable material, a known volume of the cell suspension is diluted with 0.4% trypan blue (4:1 cells to trypan blue) and viability assessed by trypan blue exclusion. Viable, non-viable and total cells are enumerated using a hemacytometer. Growth curves are constructed by plotting the number of viable cells versus the number of days in culture. Cells are shipped and implanted after reaching confluence.

For purposes of the present invention, confluence is defined as the presence of at least about 4 x 10⁵ cells/cm³ when in a flexible planar form of the implantable material (1.0 x 4.0 x 0.3 cm), and preferably about 7 x 10⁵ to 1 x 10⁶ total cells per aliquot (50-70 mg) when in a flowable composition. For both, cell viability is at least about 90% preferably but no less than 80%. If the cells are not confluent by day 12 or 13, the media is changed, and incubation is continued for an additional day. This process is continued until confluence is achieved or until 14 days post-seeding. On day 14, if the cells are not confluent, the lot is discarded. If the cells are determined to be confluent after performing in-process checks, a final media change is performed. This final media change is performed using EGM-2 without phenol red and without antibiotics. Immediately following the media change, the tubes are fitted with sterile plug seal caps for shipping.

Evaluation of Functionality and Phenotype: For purposes of the invention described herein, the implantable material is further tested for indicia of functionality and phenotype prior to implantation. For example, conditioned media are collected during the culture period to ascertain levels of heparan sulfate, transforming growth factor-β₁ (TGF-β₁), basic fibroblast growth factor (b-FGF), tissue inhibitors of matrix metalloproteinases (TIMP), and nitric oxide (NO) which are produced by the cultured endothelial cells. In certain preferred embodiments, the implantable material can be used for the purposes described herein when total cell number is at least about 2, preferably at least about 4 x 10⁵ cells/cm³ of implantable material; percentage of viable cells is at least about 80-90%, preferably ≥90%, most preferably at least about 90%; heparan sulfate in conditioned media is at least about 0.23-1.0, preferably at least about 0.5 microg/mL/day; TGF-β₁ in conditioned media is at least about 200-300 picog/mL/day, preferably at least about 300 picog/ml/day; b-FGF in conditioned media is below about 200 picog/ml, preferably no more than about 400 picog/ml; TIMP-2 in conditioned media is at least about 5.0 - 10.0 ng/mL/day, preferably at least about 8.0 ng/mL/day; NO in conditioned media is at least about 0.5 - 3.0 µmol/L/day, preferably at least about 2.0 µmol/L/day BMP-2 in conditioned media is at least about 2.5 - 25.0 pg/mL/day, preferably at least about 15.0 pg/mL/day.

Heparan sulfate levels can be quantitated using a routine dimethylmethylene blue-chondroitinase ABC digestion spectrophotometric assay. Total sulfated glycosaminoglycan (GAG) levels are determined using a dimethylmethylene blue (DMB) dye binding assay in which unknown samples are compared to a standard curve generated using known quantities of purified chondroitin sulfate diluted in collection media. Additional samples of conditioned media are mixed with chondroitinase ABC to digest chondroitin and dermatan sulfates prior to the addition of the DMB color reagent. All absorbances are determined at the maximum wavelength absorbance of the DMB dye mixed with the GAG standard, generally around 515-525 nm. The concentration of heparan sulfate per day is calculated by multiplying the percentage heparan sulfate calculated by enzymatic digestion by the total sulfated glycosaminoglycan concentration in conditioned media samples. Chondroitinase ABC activity is confirmed by digesting a sample of purified 100% chondroitin sulfate and a 50/50 mixture of purified heparan sulfate and chondroitin sulfate. Conditioned medium samples are corrected appropriately if less than 100% of the purified chondroitin sulfate is digested. Heparan sulfate levels may also be quantitated using an ELISA assay employing monoclonal antibodies.

TGF-β₁, TIMP, b-FGF, and BMP levels can be quantitated using an ELISA assay employing monoclonal or polyclonal antibodies, preferably polyclonal. Control collection media can also be quantitated using an ELISA assay and the samples corrected appropriately for TGF-β_{1,} TIMP, b-FGF and BMP levels present in control media.

Nitric oxide (NO) levels can be quantitated using a standard Griess Reaction assay. The transient and volatile nature of nitric oxide makes it unsuitable for most detection methods. However, two stable breakdown products of nitric oxide, nitrate (NO₃) and nitrite (NO₂), can be detected using routine photometric methods. The Griess Reaction assay enzymatically converts nitrate to nitrite in the presence of nitrate reductase. Nitrite is detected colorimetrically as a colored azo dye product, absorbing visible light in the range of about 540 nm. The level of nitric oxide present in the system is determined by converting all nitrate into nitrite, determining the total concentration of nitrite in the unknown samples, and then comparing the resulting concentration of nitrite to a standard curve generated using known quantities of nitrate converted to nitrite.

The earlier-described preferred inhibitory phenotype is assessed using the quantitative heparan sulfate, TGF-β₁, TIMP, NO and/or b-FGF assays described above, as well as quantitative *in vitro* assays of smooth muscle cell growth, osteoblast differentiation and survival, chondrocyte differentiation and survival, fibroblast migration and inhibition of thrombosis as follows. For purposes of the present invention, implantable material is ready for implantation when one or more of these alternative *in vitro* assays confirm that the implantable material is exhibiting the preferred regulatory phenotype.

To evaluate inhibition of smooth muscle cell growth *in vitro*, the magnitude of inhibition associated with cultured endothelial cells is determined. Porcine or human aortic smooth muscle cells are sparsely seeded in 24 or 96 well tissue culture plates in smooth muscle cell growth medium (SmCM-2, Cambrex Corp., East Rutherford, NJ). The cells arc allowed to attach for 24 hours. The media is then replaced with smooth muscle cell basal media (SmBM) containing 0.2% FBS for 48-72 hours to growth arrest the cells. Conditioned media is prepared from post-confluent endothelial cell cultures, diluted 1:1 with 2X SMC growth media and added to the cultures. A positive control for inhibition of smooth muscle cell growth is included in each assay. After three to four days, the number of cells in each sample is enumerated using a Coulter Counter or determined by colorimetric analysis after the addition of a dye. The effect of conditioned media on smooth muscle cell proliferation is determined by comparing the number of smooth muscle cells per well immediately before the addition of conditioned media with that after three to four days of exposure to conditioned media, and to control media (standard growth media with and without the addition of growth factors). The magnitude of inhibition associated with the conditioned media samples are compared to the magnitude of inhibition associated with the positive control. According to a preferred embodiment, the implantable material is considered inhibitory if the conditioned media inhibits about 20% of what the heparin control is able to inhibit.

To evaluate inhibition of thrombosis *in vitro,* the level of heparan sulfate associated with the cultured endothelial cells is determined. Heparan sulfate has both anti-proliferative and anti-thrombotic properties. Using either the routine dimethylmethylene blue-chondroitinase ABC digestion spectrophotometric assay or an ELISA assay, both assays are described in detail above, the concentration of heparan sulfate was calculated. The implantable material can be used for the purposes described herein when the heparan sulfate in the conditioned media is at least about 0.23-1.0, preferably at least about 0.5 microg/mL/day.

Another method to evaluate inhibition of thrombosis involves determining the magnitude of inhibition of platelet aggregation *in vitro* associated with platelet rich-plasma or platelet concentrate (Research Blood Components, Brighton, MA). Conditioned media was prepared from post-confluent endothelial cell cultures and added to aliquots of the platelet concentrate. A platelet aggregating agent (agonist) was added to the platelets seeded into 96 wells as control. Platelet agonists commonly include arachidonate, ADP, collagen type 1, epinephrine, thrombin (Sigma-Aldrich Co., St. Louis, MO) or ristocetin (available from Sigma-Aldrich Co., St. Louis, MO). An additional well of platelets has no platelet agonist or conditioned media added, to assess for baseline spontaneous platelet aggregation. A positive control for inhibition of platelet aggregation was also included in each assay. Exemplary positive controls include aspirin, heparin, indomethacin (Sigma-Aldrich Co., St. Louis, MO), abciximab (ReoPro^{®}. Eli Lilly, Indianapolis, IN), tirofiban (Aggrastat^{®}, Merck & Co., Inc., Whitehouse Station, NJ) or eptifibatide (Integrilin^{®}, Millennium Pharmaceuticals, Inc., Cambridge, MA). The resulting platelet aggregation of all test conditions were then measured using a plate reader and absorbance read at 405 nm. The plate reader measures platelet aggregation by monitoring optical density. As platelets aggregate, more light can pass through the specimen. The plate reader reports results in absorbance, a function of the rate at which platelets aggregate. Aggregation is assessed as maximal aggregation at 6 to 12 minutes after the addition of the agonist. The effect of conditioned media on platelet aggregation was determined by comparing maximal agonist aggregation before the addition of conditioned medium with that after exposure of platelet concentrate to conditioned medium, and to the positive control. Results are expressed as a percentage of the baseline. The magnitude of inhibition associated with the conditioned media samples are compared to the magnitude of inhibition associated with the positive control. According to a preferred embodiment, the implantable material is considered inhibitory if the conditioned media inhibits about 20% of what the positive control is able to inhibit.

When ready for implantation, the planar form of implantable material is supplied in final product containers, each preferably containing a 1 x 4 x 0.3 cm (1.2 cm³), sterile implantable material with preferably approximately 5-8 x 10⁵ or preferably at least about 4 x 10⁵ cells/cm³, and at least about 90% viable cells (for example, human aortic endothelial cells derived from a single cadaver donor) per cubic centimeter implantable material in approximately 45-60 ml, preferably about 50 ml, endothelial growth medium (for example, endothelial growth medium (EGM-2), containing no phenol red and no antibiotics). When porcine aortic endothelial cells are used, the growth medium is also EBM-2 containing no phenol red, but supplemented with 5% FBS and 50 µg/ml gentamicin.

In other preferred embodiments, the flowable composition (for example, a particulate form biocompatible matrix) is supplied in final product containers, including, for example, sealed tissue culture containers modified with filter caps or pre-loaded syringes, each preferably containing about 50-60 mg of flowable composition comprising about 7 x 10⁵ to about 1 x 10⁶ total endothelial cells in about 45-60 ml, preferably about 50 ml, growth medium per aliquot.

Shelf-Life of Implantable Material: The implantable material of the present invention comprising a confluent, near-confluent or post-confluent population of cells can be maintained at room temperature in a stable and viable condition for at least two weeks. Preferably, such implantable material is maintained in about 45-60 ml, more preferably about 50 ml per implantable material, of transport media with or without additional FBS or VEGF. Transport media comprises EGM-2 media without phenol red. FBS can be added to the volume of transport media up to about 10% FBS, or a total concentration of about 12% FBS. However, because FBS must be removed from the implantable material prior to implantation, it is preferred to limit the amount of FBS used in the transport media to reduce the length of rinse required prior to implantation. VEGF can be added to the volume of transport media up to a concentration of about 3-4 ng/mL.

Cryopreservation of Implantable Material: The implantable material of the present invention can be cryopreserved for storage and/or transport to the implantation site without diminishing its clinical potency or integrity upon eventual thaw. Preferably, implantable material is cryopreserved in a 15 ml cryovial (Nalgene^{®}, Nalge Nunc Int'l, Rochester, NY) in a solution of about 5 ml CryoStor CS-10 solution (BioLife Solutions, Oswego, NY) containing about 10% DMSO, about 2-8% Dextran and about 20-75% FBS and/or human serum. Cryovials are placed in a cold iso-propanol water bath, transferred to an -80°C freezer for 4 hours, and subsequently transferred to liquid nitrogen (-150°C to -165°C).

Cryopreserved aliquots of the implantable material are then slowly thawed at room temperature for about 15 minutes, followed by an additional approximately 15 minutes in a room temperature water bath. The material is then washed about 3 times in about 200 - 250 mL saline, lactated ringers or EBM. The three rinse procedures are conducted for about 5 minutes at room temperature. The material may then be implanted.

To determine the bioactivity of the thawed material, following the thaw and rinse procedures, the cryopreserved material is allowed to rest for about 9 to 48 hours in about 10 ml of recovery solution. For porcine endothelial cells, the recovery solution is EDM-2 supplemented with 5% FBS and 50 µg/ml gentamicin at 37°C in 5% CO₂; for human endothelial cells, the recovery solution is EGM-2 with or without antibiotics. Further post-thaw conditioning can be carried out for at least another 24 hours prior to use and/or packaging for storage or transport.

Immediately prior to implantation, the transport or cryopreservation medium is decanted and the implantable material is rinsed 2-3 times in about 250-500 ml sterile saline (USP). The medium in the final product contains a small amount of FBS to maintain cell viability during transport to a clinical site if necessary. The FBS has been tested extensively for the presence of bacteria, fungi and other viral agents according to Title 9 CFR: Animal and Animal Products. A rinsing procedure is employed just prior to implantation, which decreases the amount of FBS transferred preferably to between 0-60 ng per implant, but preferably no more than 1-2 µg per implant.

The total cell load per human patient will be preferably approximately 1.6-2.6 x 10⁴ cells per kg body weight, but no less than about 2 x 10³ and no more than about 2 x 10⁶ cells per kg body weight.

Administration of Implantable Material: The implantable material of the present invention when in a flowable composition comprises a particulate biocompatible matrix and cells, preferably endothelial cells, more preferably vascular endothelial cells, which are about 90% viable at a preferred density of about 0.8 x 10⁴ cells/mg, more preferred of about 1.5 x 10⁴ cells/mg, most preferred of about 2 x 10⁴ cells/mg, and which can produce conditioned media containing heparan sulfate at least about 0.23-1.0, preferably at least about 0.5 microg/mL/day, TGF-β₁ at at least about 200-300 picog/ml/day, preferably at least about 300 picog/ml/day, and b-FGF below about 200 picog/ml and preferably no more than about 400 picog/ml; TIMP-2 in conditioned media is at least about 5.0 - 10.0 ng/mL/day, preferably at least about 8.0 ng/mL/day; NO in conditioned media is at least about 0.5 - 3.0 µmol/L/day, preferably at least about 2.0 µmol/L/day; and, display the earlier-described inhibitory phenotype.

For purposes of the present invention generally, administration of the implantable material is localized to a site in the vicinity of, adjacent to or at a site of injury, disease, or damage of a mineralized or non-mineralized skeletal tissue such as a bone, joint, tendon, ligament and/or cartilage. The site of deposition of the implantable material is a surface of the structure. As contemplated herein, localized deposition can be accomplished as follows.

In a particularly preferred embodiment, the flowable composition is first administered percutaneously, entering the patient's body near the skeletal element and then deposited on a surface of the bone, joint, tendon, ligament, cartilage and/or other mineralized or non-mineralized skeletal tissue using a suitable needle, catheter or other suitable percutaneous delivery device. Alternatively, the flowable composition is delivered percutaneously using a needle, catheter or other suitable delivery device in conjunction with an identifying step to facilitate delivery to a desired surface of the bone, joint, tendon, ligament, cartilage and/or other mineralized or non-mineralized skeletal tissue. The identifying step can occur prior to or coincident with percutaneous delivery. The identifying step can be accomplished using physical examination, x-ray, ultrasound, and/or CT scan, to name but a few. The identifying step is optionally performed and not required to practice the methods of the present invention.

Preferably, the implantable material is deposited on a surface of a bone, joint, tendon, ligament, cartilage and/or other mineralized or non-mineralized skeletal tissue, either at the site of injury, disease or damage to be treated, or adjacent to or in the vicinity of the site of injury, disease or damage. The implantable material can be deposited in a variety of locations relative to the affected structure, for example, at the site of injury, damage or disease, surrounding the site of injury, damage or disease or adjacent to the site of injury, damage or disease. According to a preferred embodiment, an adjacent site is within about 0 mm to 20 mm of the affected skeletal element. In another preferred embodiment, a site is within about 21 mm to 40 mm; in yet another preferred embodiment, a site is within about 41 mm to 60 mm. In another preferred embodiment, a site is within about 61 mm to 100 mm. Alternatively, an adjacent site is any other clinician-determined adjacent location where the deposited composition is capable of exhibiting a desired effect on a bone, joint, tendon, ligament, cartilage and/or other mineralized or non-mineralized skeletal tissue in the proximity of the site of injury, damage or disease.

In another embodiment, the implantable material is delivered directly to a surgically-exposed surface at, adjacent to or in the vicinity of a bone, joint, tendon, ligament, cartilage and/or other mineralized or non-mineralized skeletal tissue. In this case delivery is guided and directed by direct observation of the site. Also in this case, delivery can be aided by coincident use of an identifying step as described above. Again, the identifying step is optional.

According to another embodiment of the invention, the flexible planar form of the implantable material is delivered locally to a surgically-exposed exterior site, adjacent to or in the vicinity of an injured, diseased or damaged bone, joint, tendon, ligament, cartilage and/or other mineralized or non-mineralized skeletal tissue. In one case, at least one piece of the implantable material is applied to a desired site by passing one end of the implantable material under the affected structure. The ends are then wrapped around the structure, keeping the implantable material centered. The ends overlap each other to secure the material in place. In other cases, the implantable material does not need to completely wrap around the circumference of the structure; it need only conform to and contact a surface of the structure and be implanted in an amount effective to treat an injured, damaged or diseased site.

### Examples

### 1. Bone Growth: Osteoblast Differentiation

Osteoblasts are the cells responsible for bone formation, growth of bone mass and bone repair following injury or damage to bone tissue. To evaluate regulation of osteoblasts, the ability of osteoblasts to differentiate in contact with the implantable material and/or media conditioned with the implantable material was determined. Osteoblast differentiation was evaluated in the osteoblast-like cell line MC3t3 by determining the induction of osteoblast differentiation marker gene expression levels using RT-real-time PCR analysis. MC3t3 cells will express osteoblast markers when grown in appropriate conditions, providing a useful assay system to study induction of osteoblast differentiation, and to identify potential agents for bone healing.

Media conditioned with the implantable material (VGCM) was created by incubating collection media (EBM - Phenol Red Free (Lonza Biosciences, Basel Switzerland), 0.5% fetal bovine serum (FBS, Hyclone, Logan, UT), and 0.1mg/ml Gentamicin (Biowhittaker, Walkersville, MD)) with the implantable material. Mouse osteoblast cell line MC3T3-E1 (ATCC) were seeded at 100,000 cell per well in 12-well tissue culture plates, and allowed to reach confluence in media consisting of Minimum Essential Medium Alpha Medium (αMEM, Invitrogen Corp., Carlsbad, CA) supplemented with 10% FBS, and 10µg/ml Penicillin-Streptomycin (Invitrogen).

The resulting confluent monolayers of osteoblasts were treated with 0.5-1 mL of the following: 1) control media (consisting of EBM supplemented with 0.5% FBS, 50ug/mL gentamicin, 50ug/mL ascorbic acid (Fluka, Sigma-Aldrich, St. Louis, MO) and 10 mM β-glycerolphosphate (Calbiochem, Merck KGaA, Darmstadt, Germany); 2) media conditioned with the implantable materials supplemented with 50ug/mL ascorbic acid and 10 mM β-glycerolphosphate (VGCM); or 3) for the positive control, osteoblasts were treated with BMP-2 (5 ng/mL in the control media) to induce differentiation.

At 24 hour after the addition of the different treatments, total RNA was extracted from the cells using the RNeasy Mini Column kit (Qiagen, Valencia, CA) and used to generate cDNA utilizing the SuperScript III First Strand Synthesis System for RT-PCR (Invitrogen). Using the resulting cDNA, the expression levels of 2 osteoblast differentiation marker genes, osteopontin and bone sialoprotein, by each treatment group of osteoblasts were determined by real-time PCR (iQ5, Bio-Rad, Hercules, CA).

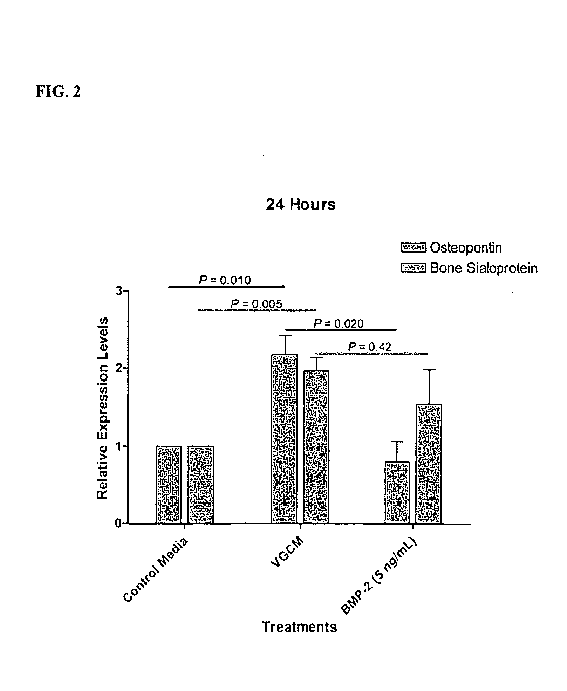

Figure 2 depicts the relative expression levels of osteopontin and bone sialoprotein in osteoblasts at 24 hours post treatment according to the osteoblast differentiation assay described above. At 24 hours, MC3t3 osteoblasts incubated with media conditioned with the implantable material exhibited about 2-fold increase in the detectable levels of osteopontin and bone sialoprotein expression compared to control. However, the osteogenic growth factor BMP-2 treatment did not induce any detectable increase in the expression levels of osteopontin and only about 1.5 fold increase in bone sialoprotein expression compared to control. The results suggested that media conditioned by the implantable materials contains osteogenic induction activities as indicated by the induction of osteopontin and bone sialoprotein in the osteoblasts, and the osteogenic activities of the implantable material are also more potent than BMP-2 at 5 ng/mL for inducing osteoblast differentiation. Administration of the implantable material to the site of bone injury or damage is believed to result in improved bone formation and bone mass growth, contributing to healing of the injured or damaged bone region compared to control. Accordingly, administration of the implantable material to a site of bone injury or damage in an individual in need will improve the healing response including improved healing time, bone junction formation and bone accumulation at the site of injury or damage and contribute to an enhanced therapeutic response to the injury or damage in the treated individual.

### 2. Bone Growth: Osteoblast Survival

Osteoblasts are the cells responsible for bone formation, growth of bone mass and bone repair following injury or damage to bone tissue. To evaluate regulation of osteoblasts, the magnitude of osteoblast survival in contact with the implantable material and/or media conditioned with the implantable material was determined. Mouse osteoblasts from cell line MC3T3-E1 (ATCC) were seeded in 12 well tissue culture plates to a density of 10,000 cell/well in assay media consisting of Minimum Essential Medium Alpha Medium (αMEM, Invitrogen) supplemented with 10% fetal bovine serum (FBS, Hyclone) and 10µg/ml Penicillin-Streptomycin (Invitrogen). The cells were differentiated for 14 days, at which time the resulting confluent monolayers of osteoblasts were washed and medium changed to collection medium (EBM without phenol red and with 0.5% FBS). The implantable material was then added to culture inserts which were incubated about the wells containing the osteoblasts. Inserts containing no material were used as control. Tumor Necrosis Factor alpha (TNFα) (10 ng/ml) was added into the co-culture wells. After 24 hours, treated cultures and untreated controls were then incubated with Trypan blue 20%. The magnitude of osteoblast survival associated with the implantable material was compared to the magnitude of osteoblast survival associated with the addition of TNFα without the implantable material and to control wells without TNFα.

Photomicrographs of mouse osteoblasts stained with Trypan blue according to the osteoblast survival assay described above were obtained. According to one embodiment, images of Trypan blue stained osteoblasts were taken at 40X magnification using a Nikon phase microscope and a Nikon D40 camera. The effect of the implantable material on osteoblast survival was determined by comparing the amount of blue-staining present with TNFα compared to the implantable material and the negative control. According to a preferred embodiment, the implantable material is considered to have a positive effect on osteoblast survival if the implantable material results in a decrease in Trypan blue staining of about 20% compared to control TNFα treatment. Alternatively, osteoblast survival can also be evaluated by the use of a colorimetric dye (Promega, Madison, WI) for determining the number of viable cells or cytotoxicity. A dye is added to the cultures followed by incubation at 37° C for 2 hours. Absorbance is read at 490 nm. The magnitude of absorbance correlates to cell viability.

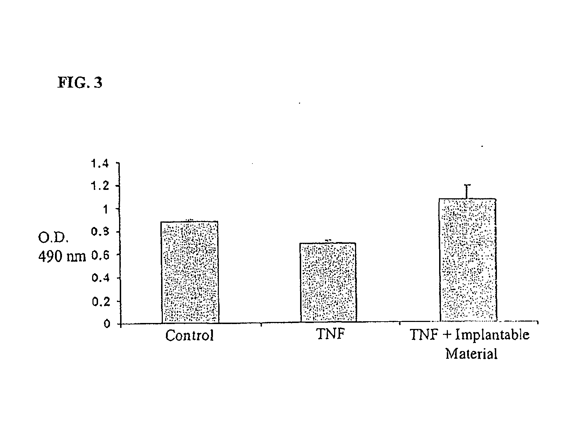

Figure 3 is a graphical representation of the addition of a colorimetric dye to mouse osteoblasts with and without TNFα or the implantable material. According to a preferred embodiment, the implantable material is considered to have a positive effect on osteoblast survival if the implantable material results in an increase in absorbance of about 20% compared to control TNFα treatment. The dosage of TNFα to an individual well can also be evaluated at each of, for example, 0 picog/ml, 100 picog/ml, 200 picog/ml, 400 picog/ml, 600 picog/ml, 800 picog/ml and 1000 picog/ml. Alternatively, the exposure time to TNFα can be evaluated at each of 1 hr, 4 hrs, 12 hrs, 24 hrs, 48 hrs, and 96 hrs. As an alternate embodiment, cell survival can also be evaluated after serum starvation without the addition of TNFα. According to one embodiment, the treated cells are differentiated cells. According to another embodiment, the treated cells are non-differentiated cells.

Administration of the implantable material to the site of bone injury or damage is believed to result in improved bone formation and bone mass growth, contributing to healing of the injured or damaged bone region compared to control. Accordingly, administration of the implantable material to a site of bone injury or damage in an individual in need will improve the healing response including improved healing time, bone junction formation and bone accumulation at the site of injury or damage and contribute to an enhanced therapeutic response to the injury or damage in the treated individual.

### 3. Cartilege Growth: Chondrocyte Survival

Chondrocytes are the primary cells found in cartilage that are responsible for the production, repair and maintenance of the cartilaginous matrix. To evaluate regulation of chondrocytes, the magnitude of chondrocyte survival in contact with the implantable material and/or media conditioned with the implantable material was determined. Mouse chondrocytes from cell line ATDC5 were seeded in 12 well tissue culture plates to a density of 10,000 cell/well in assay media consisting of Minimum Essential Medium/F12 Medium (MEM/F12, Invitrogen) supplemented with 5% fetal bovine serum (FBS, Hyclone) and 10µg/ml Penicillin-Streptomycin (Invitrogen). The cells were differentiated for 14 days, at which time the resulting confluent monolayers of chondrocytes were washed and medium changed to collection medium (EBM without phenol red and with 0.5% FBS). The implantable material was added to culture inserts which were incubated above the wells containing the chondrocytes. Inserts containing no material were used as control. Tumor Necrosis Factor alpha (TNFα) (10 ng/ml) was added into the co-culture wells. After 24 hours, treated cultures and untreated controls were incubated with Trypan blue 20%. The magnitude ofchondrocyte survival associated with the implantable material was compared to the magnitude of chondrocyte survival associated with the addition of TNFα without the implantable material and to control wells containing no TNFα.

Photomicrographs of mouse chondrocytes stained with Trypan blue according to the chondrocyte survival assay described above were obtained. According to one embodiment, images of Trypan blue stained chondrocytes were taken at 40X magnification using a Nikon phase microscope and a Nikon D40 camera. The effect of the implantable material on chondrocyte survival was determined by comparing the amount of blue-staining present with TNFα compared to the implantable material and the negative control. According to a preferred embodiment, the implantable material is considered to have a positive effect on chondrocyte survival if the implantable material results in a decrease in Trypan blue staining of about 20% compared to control TNFα treatment. Alternatively, chondrocyte survival can also be evaluated by the use of a colorimetric dye (Promega) for determining the number of viable cells or cytotoxicity. A dye was added to the culture followed by 2 hour incubation at 37°C. Absorbance was read at 490 nm. The magnitude of absorbance correlates to cell viability.

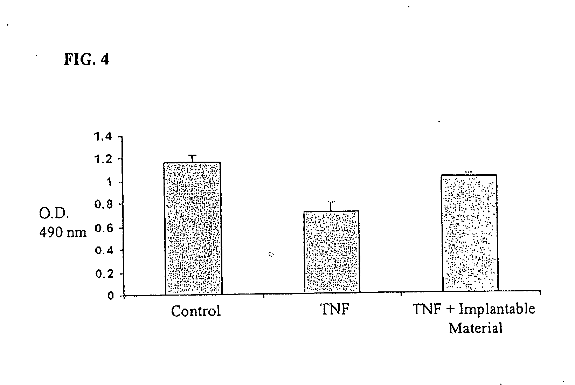

Figure 4 is a graphical representation of the addition of a colorimetric dye to mouse chondrocytes with and without TNFα or the implantable material. According to a preferred embodiment, the implantable material is considered to have a positive effect on chondrocyte survival if the implantable material results in an increase in absorbance of about 20% compared to control TNFα treatment.

The dosage of TNFα to an individual well can also be evaluated at each of, for example, 0 picog/ml, 100 picog/ml, 200 picog/ml, 400 picog/ml, 600 picog/ml, 800 picog/ml and 1000 picog/ml. Alternatively, the exposure time to TNFα can be evaluated at each of 1 hr, 4 hrs, 12 hrs, 24 hrs, 48 hrs, and 96 hrs. As an alternate embodiment, cell survival can also be evaluated after serum starvation without the addition of TNFα. According to one embodiment, the treated cells are differentiated cells. According to another embodiment, the treated cells are non-differentiated cells.

Administration of the implantable material to the site of cartilage injury or damage is believed to result in improved cartilage formation, contributing to healing of the injured or damaged cartilage region compared to control. Accordingly, administration of the implantable material to a site of cartilage injury or damage in an individual in need will improve the healing response including improved healing time, cartilage formation and cartilage accumulation at the site of injury or damage and contribute to an enhanced therapeutic response to the injury or damage in the treated individual.

### 4. Cytokine Mediated Cartilage Damage

Chondrocytes are the primary cells found in cartilage that are responsible for the production, repair and maintenance of the cartilaginous matrix. Plugs of porcine articular cartilage can be utilized to evaluate whether the implantable material can prevent or diminish cytokine mediated cartilage damage and/or accelerate recovery from cytokine mediated cartilage damage. The cytokine interleukin-lalpha (IL-1α) can induce glycosaminoglycan (GAG) breakdown and, therefore, cartilage damage or degradation. Cartilage degradation can be measured by the GAG content of the culture media, allowing the evaluation of any potential chondroprotective effects *in vitro.* This assay system was utilized to evaluate potential chondroprotective properties of the implantable materials and/or the media conditioned by the implantable material.

To evaluate the chondroprotective activities of the implantable material, the ability of the media conditioned by the implantable materials (VGCM) to protect against the damaging effects of IL-1α towards the cartilage plug, as well as to promote faster recovery of the cartilage post-IL-1α treatment was determined.

Porcine articular cartilage plugs were obtained from pig knee joints using a #2 cork bore (∼6mm diameter). Cartilage plugs were washed thoroughly in DMEM medium (Invitrogen) supplemented with penicillin and streptomycin (Invitrogen), incubated in the same media at 37°C with 5% CO₂ for 12-16 hours, and then trimmed using a 6x1 mm template to allow for uniform samples while removing excess tissue and mineralized bone from the cartilage plugs. Trimmed cartilage plugs were washed and incubated for 24-72 hours at 37°C with 5% CO₂.

The cartilage plugs were placed individually into wells of a 24-well plate for the chondroprotective studies and incubated with: 1) control media (EBM-2 supplemented with 0.5% FBS and 50 ug/mL ascorbic acid without IL-1α) for 12 days ("Control Media"); 2) 10 ng/mL IL-1α (PeproTech, Rocky Hill, NJ) in Control Media for the initial 3 days, followed by additional 9 days in Control Media only (IL-1α); 3) media conditioned by the implantable materials ("VGCM") plus 10ng/mL IL-1α for the initial 3 days, followed by VGCM only for another 9 days (VGCM+IL-1α); 4) 10 ng/mL IL-1α and TGF-β1 in Control Media for the initial 3 days, followed by 10 ng/mL TGF-β1 in Control Media for another 9 days (TGF-β1+IL-1α); 5) 10 ng/mL IL-1α in Control Media for the initial 3 days, followed by VGCM only for another 9 days (IL-1α/VGCM); or 6) 10 ng/mL IL-1α in Control Media for the initial 3 days, followed by 10 ng/mL TGF-β1 in Control Media for another 9 days (IL- 1α/TGF-β1).

In all conditions, media samples were collected and replaced with fresh media every 48-72 hours. The media samples collected at each time point were used for GAG analysis in order to determine the status of the cartilage plugs. At the conclusion of the experiment, the individual plugs were weighed. The weights of the cartilage plugs were used for data normalization in the final calculation of GAG release (expressed as µg of GAG release/mg of tissue).

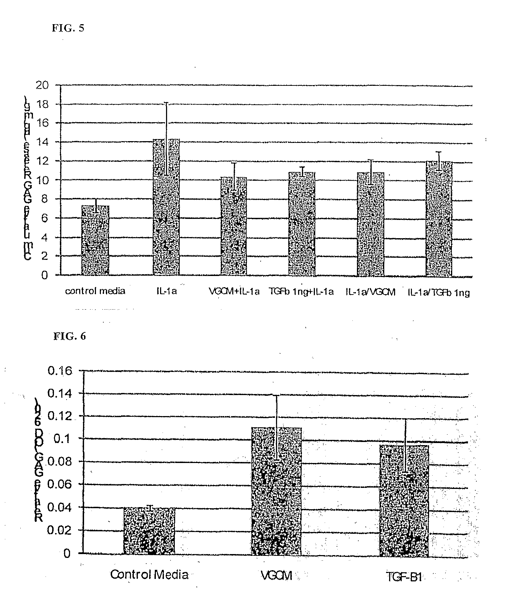

] Figure 5 is a graphical representation of suppression of IL-1α-mediated cartilage damage (lower GAG release) by media conditioned by the implantable material (VGCM). There was a significant increase in GAG release or cartilage damage when porcine articular cartilage plugs were incubated with IL-1α compared to Control Media. Concurrent incubation with media conditioned by the implantable materials (VGCM+IL-1α) decrease the total GAG loss by 27% at the end of the experiment. Similarly, concurrent incubation with TGF-β1 (TGF-β1+IL-1α) decreased IL-1α-induced GAG loss by 24%. A similar trend was observed when VGCM and TGF-β1 were added to porcine articular cartilage plugs after the initial 3 day of IL-1α incubation. Incubation with VGCM post IL-1α treatment (IL-1α/VGCM) decreased GAG release by 24%, while the addition of TGF-β1 (IL-1α/TGF-β1) decreased GAG release by 15% compared to the IL-1α only treatment.

These data indicate that incubation with VGCM can diminish the IL-1α-mediated cartilage damage, demonstrating the chondroprotective function of the implantable materials, which is comparable to, if not better than, that provided by TGF-β1, a well-established growth factor with chondroprotective activities. Administration of the implantable material to the site of cartilage injury or damage is believed to result in improved cartilage formation and protection, contributing to maintenance and healing of the injured or damaged cartilage region compared to control. Accordingly, administration of the implantable material to a site of cartilage injury or damage in an individual in need will improve the healing response, including improved healing time, cartilage formation and cartilage accumulation at the site of injury or damage and contribute to an enhanced therapeutic response to the injury or damage in the treated individual.

### 5, Cartilege Repair: Chondrocyte Synthesis of ECM Components

Chondrocytes are the primary cells found in cartilage that are responsible for the production, repair and maintenance of the cartilaginous matrix. Primary porcine anicular chondrocytes can produce and accumulate extracellular matrix (ECM) components during culture, providing an assay to evaluate the function of the implantable material in promoting the synthesis of cartilage-specific ECM components, such as glycosaminoglycans (GAGs), and therefore cartilage repair.

To evaluate cartilage repair, the function of media conditioned by the implantable materials (VGCM) in promoting GAGs production and accumulation by primary articular chondrocytes was determined. Media conditioned by the implantable material (VGCM) was created by incubating collection media (EBM - Phenol Red Free (Lonza), 0.5% FBS (Hyclone), and 0.1 mg/ml Gentamicin (Lonza)) with the implantable material. To isolate the primary chondrocytes, slices of articular cartilage from porcine knee joints were harvested and washed thoroughly with DMEM medium (Invitrogen) supplemented with penicillin/streptomycin (Invitrogen).

Cartilage slices were incubated at 37°C with 5% CO₂ in a tissue culture incubator for 12-16 hours, washed with the same DMEM medium and digested with collagenase (1-3mg/mL, Sigma) for 48-72 hours at 37°C to obtain the articular chondrocytes. The isolated chondrocytes were filtered through a sterile nylon mesh (70 µm, BD Biosciences, San Jose, CA), and were seeded in 24 well plates at about 100,000 cells/cm² in DMEM medium supplemented with penicillin/streptomycin and 10% FBS. The primary chondrocytes were allowed to attach for several days with media changes every 2-3 days thereafter. On day 10 of culture, chondrocytes were washed with serum-free DMEM or DPBS (Invitrogen) and incubated with: 1) Control Medium; 2) VGCM; or 3) 1 ng/mL TGF-β1 in Control Medium.

Control Medium was EBM-2 supplemented with 0.5% FBS, 50 ug/mL ascorbic acid (Sigma) and 50 ug/mL gentamicin (Biowhittaker). The primary chondrocytes were incubated with the various media for 4 days, and then washed with DPBS, fixed with 4% paraformaldehyde (Electron Microscope Systems, Inc., Hatfield, PA) for 30 minutes at room temperature, then stained for GAGs with a solution of alcian blue (1% in 0.1M HCl, Sigma) for 2-6 hours. The stained chondrocyte cell layers were washed extensively with water to remove excess alcian blue stain. After photographing, the alcian blue was extracted from the cell layers with 8M GuHCl (Pierce Protein Research Products, Thermo Fisher Scientific, Waltham, MA) for 3-12 hours. The amount of alcian blue extracted from the chondrocyte cell layer was proportional to the amounts of GAGs produced and accumulated by the cells, and was determined by measuring the absorbance of the extracted alcian blue samples at 620 nm in a plate reader (MultiSkan Spectrum, Thermo Fisher Scientific).

] Figure 6 is a graphical representation of enhanced GAG production and accumulation by primary porcine chondrocytes after treatment with the implantable material. There was a significant increase in alcian blue staining and thus GAG production and accumulation in chondrocyte samples treated with VGCM (P<0.003) compared to Control Medium. Incubation with TGF-β1 also significantly increased GAG production and accumulation by the chondrocytes (*P*<0.003), suggesting that TGF-β1 can induce ECM synthesis by chondrocytes. Interestingly, the VGCM has comparable, if not greater cartilage-specific GAG induction function as 1 ng/mL of TGF-β1, suggesting that VGCM may be even more potent in promoting cartilage repair than using a single growth factor such as TGF-β1.

These data indicate that VGCM can promote the synthesis of cartilage-specific ECM components (GAG) by primary articular chondrocyte and therefore are believed to promote cartilage repair *in vivo.* Accordingly, administration of the implantable material to a site of cartilage injury or damage in an individual in need will improve the healing response, including improved healing time, cartilage formation and cartilage accumulation at the site of injury or damage and contribute to an enhanced therapeutic response to the injury or damage in the treated individual.

### 6. Repair of Closed Fractures.

The closed fracture rat model described by Diwan et al. (J. Bone Miner. Res., 2000 Feb;15(2):342-51) will be studied to demonstrate treatment and management of closed fractures. Closed femoral fractures will be created in rats by three point bending. Two groups of animals will be maintained similarly, except the treatment group will receive an effective amount of the flowable formulation of the implantable material by percutaneous injection at or near the fracture site. Reduction of the fracture in both groups will be performed by casting or other appropriate procedure. Bone healing will be monitored over time by X-ray, and/or by sacrificing the animals and visually examining the fracture. It is expected that rats treated with the implantable material will display improved bone healing over the control group.

### 7. Surgical Repair of Open Fractures.

The open fracture rat model described by Diwan et al. (J. Bone Miner. Res., 2000 Feb; 15(2):342-51) will be studied to demonstrate treatment and management of open fractures. Open femoral fractures will be created in rats by surgical procedure to sever tissue near the femur and create a fracture in the femur with a gigli saw. Two groups of animals will be maintained similarly, except the treatment group will receive an effective amount of the implantable material at or near the fracture site during surgical reduction of the fracture. Reduction of the fracture in both groups will be performed by surgical procedures. Bone healing will be monitored over time by X-ray, and/or by sacrificing the animals and visually examining the fracture. It is expected that rats treated with the implantable material will display improved bone healing over the control group.

### 8. Growth and Differentiation of Bones in Culture

A mouse embryonic tibiae culture model described by Agoston et al. and Serra et. al (BMC Dev Biol. 2007 Mar 20;7:18; J Cell Biol. 1999 May 17;145(4):783-94) will be used to demonstrate growth and differentiation of bone treated with the implantable material. On day 0, tibiae from 15 day embryos in CD I timed-pregnant mice will be isolated under a stereomicroscope. Tibiae will be allowed to recover from dissection overnight in serum-free α-MEM media containing 0.2% Bovine Serum Albumin (BSA), 0.5 mM L-glutamine, 40 units penicillin/mL and 40 µg streptomycin/mL as described. The following morning, bones in 24-well Falcon plates will be measured using an eyepiece in a Stemi DV4 Stereomicroscope and placed in a netwell 12 well plate and treated with the implantable material or conditioned media from the implantable material. Control bones will be included that are maintained similarly but not treated with the implantable material. Media will be changed every 48 hrs beginning on day 1. To determine growth, bones will be measured on days 1, 3, 6, and 8. It is expected that bones treated with the implantable material will display increased length relative to the controls. For weight determination and Alizarin Red/Alcian Blue staining, bones will be weighed at day 6 and then placed in 4% Paraformaldehyde (PFA) in DEPC-treated PBS for overnight fixation. Subsequently, tibiae will be placed in staining solution for 45-60 minutes (0.05% Alizarin Red, 0.015% Alcian Blue, 5% acetic acid in 70% ethanol). Images of stained bones will be taken. It is expected that the bones treated with the implantable material will show increased differentiation over the controls.

### 9. Tendon-to-Bone Healing

The rabbit ACL reconstruction model described by Kohno et al. (J. Orthop. Sci., 2007; 12:67-73) will be studied to demonstrate treatment and management of tendon-to-bone healing. The proximal extensor digitorum longus (EDL) tendon will be detached in rabbits and passed through tibial and femoral bone tunnels adjacent to the ACL and the posterior cruciate ligament. The graft will then be fixed to the bone by appropriate methods. Two groups of animals will be maintained similarly, except the treatment group will receive an effective amount of the implantable material at or near the reconstruction site during the surgical procedure. Tendon-to-bone healing will be monitored over time by MRI, physical exam, or by sacrificing the animals and visually examining the graft. It is expected that rabbits treated with the implantable material will display improved tendon-to-bone healing over the control group.

### 10. Repair of Damaged Cartilage.

The rat model described by Moore et al. (Osteoarthritis and Cartilage, 2005 13:623-631) will be studied to demonstrate treatment and management of cartilage injury, damage or disease. Cartilage injury be induced in rats by surgically making a full-thickness cut of the meniscus. Two groups of animals will be maintained similarly, except the treatment group will receive an effective amount of the implantable material at or near the meniscal tear site by injection or during the surgical procedure. The ability of the meniscus to heal will be monitored over time by MRI, or by sacrificing the animals and visually examining the meniscus. It is expected that rats treated with the implantable material will display improved meniscal healing over the control group.

### 11. Treatment of human patients with bone disorders.

Human patients that have been diagnosed with injury, damage or disease to a bone, joint, tendon, ligament, cartilage and/or other mineralized or non-mineralized skeletal tissue will be studied to demonstrate treatment or management of bone disorders. Patients will be examined to identify an affected skeletal element. Two groups of patients will be maintained similarly, except one group will receive an effective amount of the implantable material at or near the injured, damaged or diseased structure. Reduction and/or amelioration of injury or disease of the affected skeletal element will be monitored over time by ultrasound, MRI, X-ray, physical exam, and other relevant procedures depending on the type of disorder present in the patient. It is expected that patients treated with the implantable material will display reduction and/or amelioration of injury, damage or disease of the affected skeletal element.

### 12. Surgical treatment of human patients with bone disorders.

Human patients that have been diagnosed with injury, damage or disease to a bone, joint, tendon, ligament, cartilage and/or other mineralized or non-mineralized skeletal tissue and who will be undergoing surgery for those disorders will be studied to demonstrate treatment and management of these disorders. Patients will be examined to determine the affected skeletal element and effective surgical treatment. Two groups of patients will be maintained similarly, except the treatment group will receive an effective amount of the implantable material in conjunction with surgery of the injured, damaged or diseased structure. Reduction and/or amelioration of injury or disease of the affected structure will be monitored over time by ultrasound, MRI, X-ray, physical exam, and other relevant procedures depending on the type of bone disorder present in the patient. It is expected that patients treated with the implantable material will display reduction and/or amelioration of the injury, damage or disease of the affected structure at a higher level than the control group.

The invention may be embodied in other specific forms without departing from the spirit or essential characteristics thereof. The present embodiments are therefore to be considered illustrative and not restrictive, the scope of the invention being indicated by the appended claims rather than by the foregoing description, and all changes which come within the meaning and range of equivalency of the claims are therefore intended to be embraced therein.

### Embodiments

Embodiment 1: A method of treating a skeletal system disorder in an individual in need thereof, the method comprising: contacting with an implantable material a surface of a skeletal element at or adjacent to or in the vicinity of an area of injury, damage or disease, wherein said implantable material comprises a biocompatible matrix and cells and further wherein said implantable material is in an amount effective to treat the skeletal system disorder in said individual.
Embodiment 2: The method of embodiment 1 wherein the'skeletal system disorder is: (a) a bone fracture; (b) osteoporosis; (c) Paget's disease of the bone; (d) rheumatoid arthritis; (e) osteoarthritis; (f) a nonunion fracture; (g) a damaged ligament; (h) a damaged tendon; (i) a tendon graft; (j) a ligament graft; (k) a bone graft or (l) a cartilage injury.
Embodiment 3: The method of embodiment 1 wherein the skeletal element is a bone, tendon, ligament, cartilage, joint, mineralized skeletal tissue, or non-mineralized skeletal tissue.
Embodiment 4: The method of embodiment 1 wherein the biocompatible matrix is a flexible planar material or a flowable composition.
Embodiment 5: The method of embodiment 1 wherein the cells are endothelial, epithelial, endothelial-like, epithelial-like, non-endothelial cells or analogs thereof.
Embodiment 6: The method of embodiment 1 wherein the cells comprise a co-culture of at least two different cell types.
Embodiment 7: The method of embodiment 1 wherein the implantable material promotes: (a) bone repair; (b) tendon repair; (c) ligament repair; (d) cartilage repair; (e) osteoblast differentiation and/or survival; or (f) chondrocyte differentiation and/or survival.
Embodiment 8. A method of treating a skeletal element in an individual in need thereof, the method comprising: contacting with an implantable material a surface of a skeletal element at or adjacent to or in the vicinity of an area of surgical intervention, wherein said implantable material comprises a biocompatible matrix and cells and further wherein said implantable material is in an amount effective to treat the skeletal element in said individual.
Embodiment 9: The method of embodiment 8 wherein the skeletal element is as defined in embodiment 3.
Embodiment 10: The method of embodiment 9 wherein the biocompatible matrix is as defined in embodiment 4.
Embodiment 11: The method of embodiment 9 wherein the cells are as defined in embodiment 5 or embodiment 6.
Embodiment 12: The method of embodiment 9 wherein the implantable material is as defined in embodiment 7.
Embodiment 13: A composition suitable for the treatment or management of an injured or damaged skeletal element, the composition comprising a biocompatible matrix and cells, wherein said composition is in an amount effective to treat or manage the injured or damaged skeletal element.
Embodiment 14: The composition of embodiment 13 wherein the skeletal element is as defined in embodiment 3.
Embodiment 15: The composition of embodiment 13 wherein the biocompatible matrix is as defined in embodiment 4.
Embodiment 16: The composition of embodiment 13 wherein the flowable composition further comprises an attachment peptide and the cells are engrafted on or to the attachment peptide.
Embodiment 17: The composition of embodiment 13 wherein the cells are as defined in embodiment 5 or embodiment 6.
Embodiment 18: The composition of embodiment 13 wherein the cells comprise a population of cells selected from the group consisting of near-confluent cells, confluent cells and post- confluent cells.
Embodiment 19: The composition of embodiment 13 wherein the cells are at least about 80% viable.
Embodiment 20: The composition of embodiment 13 wherein the cells are not exponentially growing cells.
Embodiment 21: The composition of embodiment 13 wherein the cells are engrafted to the biocompatible matrix via cell to matrix interactions.
Embodiment 22: The composition of embodiment 13 wherein the composition further comprises a second therapeutic agent.
Embodiment 23: The composition of embodiment 13 wherein the composition further comprises: (a) an agent that inhibits infection; (b) an anti-inflammatory agent; (c) an attachment peptide; or (d) a TGF-β, a BMP, a CDMP and/or a GDF.

## Claims

1. A composition suitable for the treatment or management of an injured or damaged skeletal element, the composition comprising a biocompatible matrix and anchored or embedded cells comprisiing: (a) endothelial cells or endothelial-like cells; and (b) non-endothelial cells, said cells being present in an amount effective to treat or manage the injured or damaged skeletal element.

2. The composition of claim 1 wherein the non-endothelial cells are selected from:
epithelial cells, osteoblasts, osteocytes, osteoclasts, fibroblasts, tenocytes, ligament cells, chondrocytes, secretory cells, smooth muscle cells, stem cells, bone stem cells, endothelial progenitor cells, cardiomyocytes, secretory and ciliated cells.

3. The composition of claim 1 or claim 2 for use in a method of treating a skeletal system disorder by administering said composition to a skeletal element at or adjacent to or in the vicinity of an area of injury, damage, disease or surgical intervention.

4. The composition of claim 3 wherein the skeletal system disorder is selected from:
a bone fracture; osteoporosis; Paget's disease of the bone; rheumatoid arthritis; osteoarthritis; a nonunion fracture; a damaged ligament; a damaged tendon; a tendon graft; a ligament graft; a bone graft and a cartilage injury.

5. The composition of claim 3 wherein the skeletal element is selected from: a bone; a tendon; a ligament; cartilage; a joint; mineralized skeletal tissue and non-mineralized skeletal tissue.

6. The composition of any one of the preceding claims wherein the biocompatible matrix is a flexible planar material.

7. The composition of any one of claims 1 to 5 wherein the biocompatible matrix is a flowable composition.

8. The composition of any one of the preceding claims wherein the cells comprise a co-culture of at least two different cell types.

9. The composition of any one of the preceding claims which promotes bone repair, tendon repair, ligament repair, cartilage repair, osteoblast differentiation and/or survival or chondrocyte differentiation and/or survival.

10. The composition of claim 3 wherein said method of treating a skeletal system disorder further comprises inducing extracellular matrix production by primary articular chondrocytes.

11. The composition of claim 3 wherein said method of treating a skeletal system disorder further comprises chondroprotection.

12. The composition of claim 3 wherein said method of treating a skeletal system disorder further comprises promoting osteoblast and/or chondrocyte survival.

13. The composition of claim 1 wherein said method of treating a skeletal system disorder further comprises osteogenic induction.
